(19) Europäisches Patentamt
European Patent Office
Office européen des brevets

(11) Publication number: 0 271 979 A1

# (12) EUROPEAN PATENT APPLICATION

(21) Application number: 87309068.2

(22) Date of filing: 14.10.87

(51) Int. Cl.⁴: B01J 13/02 , C08J 3/02 , C08J 3/28 , C08L 83/04

(30) Priority: 17.12.86 US 942742

(43) Date of publication of application: 22.06.88 Bulletin 88/25

(84) Designated Contracting States: CH DE FR GB IT LI NL

(71) Applicant: DOW CORNING CORPORATION
3091 S. Saginaw Road
Midland Michigan 48686-0995(US)

(72) Inventor: Yin Kwai Lo, Peter
3109 Camberly Lane
Midland, Michigan(US)
Inventor: Ziemelis, Maris Jazeps
2015 W. Nelson
Midland, Michigan(US)

(74) Representative: Kyle, Diana et al
ELKINGTON AND FIFE High Holborn House
52/54 High Holborn
London WC1V 6SH(GB)

## (54) Process for preparing silicone microparticles.

(57) Microparticles, such as microspheres and microcapsules, comprising a solid organopolysiloxane are prepared by curing a dispersion of discrete entities by means of ultraviolet radiation. The discrete entities are dispersed in a fluid continuous phase and are sphere-like particles of a curable liquid organopolysiloxane which contains reactive acryloxy, methacryloxy or acrylamide functionality, or such a liquid organopolysiloxane containing a material to be encapsulated. The microparticles may be elastomeric or resinous and are useful as filler particles and time-release capsules.

EP 0 271 979 A1

Xerox Copy Centre

# PROCESS FOR PREPARING SILICONE MICROPARTICLES

This invention relates to a process for preparing microparticles comprising a solid organopolysiloxane. More particularly, this invention relates to the preparation of microspheres and microcapsules using ultraviolet radiation to convert dispersed discrete entities of a liquid composition consisting essentially of an organopolysiloxane having acryloxy, methacryloxy or acrylamide functionality (hereinafter acryl-functional siloxane) to the solid state.

Acryl-functional silicone compounds are known. Such materials are, for example, disclosed by Lee and Lutz in copending application Serial No. 816,477 filed January 6, 1986, and assigned to the assignee of this invention. In this case, acryl silane (or silicone) compounds are made by reacting an amine-functional silane (or amine-functional silicone) with a di-or multi-functional acryl compound by a Michael addition reaction. These acryl silane (silicone) compounds are said to be purer than those formed by other routes since no catalyst is used and no by-products are formed. The compounds are further said to have utility as adhesion promoters (silanes) and as coating compositions which can be cured by ultraviolet radiation (silicones).

Japanese Patent No. 52-43779 provides a method for producing microcapsules using a photosensitive resin and ultraviolet radiation. The photosensitive resin comprises a monomer or oligomer having two or more photosensitive groups which will undergo a polymerization reaction under the influence of light energy. Examples of photosensitive groups disclosed therein, which undergo said polymerization reaction, include acryloyl, vinyl ether, Vinyl thioether, vinyl ether, vinyl linked to benzene, N.N-vinylalkylamino, allyl, acrylamide, 1,2-alkylene oxid and acetylenyl.

Ziemelis, in United States Patent No. 4,370,160 teaches a process for preparing microspheres of solid organopolysiloxane or microcapsules which consist essentially of an internal material dispersed throughout a solid organopolysiloxane. In brief, this process comprises irradiating a dispersion of discrete entities with ultraviolet (UV) radiation. The discrete entities are dispersed in a UV-transparent fluid continuous phase and are sphere-like particles of a UV-curable, liquid organopolysiloxane composition which is immiscible with the continuous phase, or such a liquid organopolysiloxane composition containing a material to be encapsulated. The microparticles may be elastomeric or resinous and are useful as filler particles and time-release capsules. The liquid organopolysiloxane composition, in turn, consists essentially of a mixture of an organopolysiloxane having at least two vinyl (or butenylene) radicals per molecule and an organopolysiloxane having at least two mercaptoalkyl (or silicon-bonded hydrogen) radicals per molecule.

The process taught by Ziemelis, cited supra, requires a mixture of two organopolysiloxanes. It has now been found that certain organopolysiloxanes containing acryloxy, methacryloxy or acrylamide functionality may be readily dispersed in an ultraviolet transparent continuous fluid phase using conventional surfactants. Upon exposure to ultraviolet radiation, these compositions, containing a single organopolysiloxane component, cure to form solid microparticles or microcapsules.

The present invention therefore relates to a process for preparing microspheres of solid organopolysiloxane, said process comprising:

(I) preparing a dispersion of discrete entities in a fluid continuous phase by dispersing, in the continuous phase fluid, a liquid organopolysiloxane composition, convertible by ultraviolet radiation to the solid state, said fluid continuous phase being transparent to ultraviolet radiation and said liquid organopolysiloxane composition being insoluble in the fluid continuous phase and consisting essentially of an organopolysiloxane having attached thereto through silicon-carbon bonds an average of at least two Z groups per molecule, wherein Z is a monovalent organic moiety containing at least one acryl group selected from acryloxy, methacryloxy or acrylamide groups, said organopolysiloxane having an average of more than two of said acryl groups per molecule; and

(II) exposing the dispersion of (I) to ultraviolet radiation until the liquid organopolysiloxane composition is converted to the solid state.

This invention further relates to a similar process for preparing microcapsules of an internal material localized as a core in a solid organopolysiloxane. This process involves dispersing the internal material in the continuous phase fluid and simultaneously or subsequently codispersing therewith the liquid organopolysiloxane.

This invention still further relates to a similar process for preparing microcapsules of an internal material dispersed throughout a solid organopolysiloxane. This process involves dispersing or dissolving the internal material in the liquid organopolysiloxane composition before it is dispersed in the continous phase fluid.

Microparticles, as used herein, is a generic term and includes microspheres and microcapsules comprising a solid organopolysiloxane. Microspheres, as used herein, are homogeneous microparticles consisting essentially of solid organopolysiloxane. Microcapsules, as used herein, are homogeneous or

heterogeneous microparticles consisting essentially of an internal material which is different from and surrounded by the solid organopolysiloxane. Microcapsules may contain the internal (i.e., encapsulated) material dispersed throughout, or localized as a core in the solid organopolysiloxane. Microparticles, for the purposes of this invention, are essentially sphere-like particles having a diameter of up to about 5 mm, but preferably from 0.005 to 1 mm.

Liquid organopolysiloxane compositions convertible to the solid state which are suitable in the process of this invention must experience a change to the solid, i.e., non-flowing, state when cured by exposure to ultraviolet radiation (i.e., electromagnetic radiation of wavelength in the range of 200 to 400 nm). Compositions meeting this requirement comprise a liquid organopolysiloxane bearing an average of more than two acryl radicals per molecule. The term "acryl" as used herein denotes a generic representation of acryloxy, methacryloxy or acrylamide functionalities.

The liquid organopolysiloxane compositions convertible to the solid state which are suitable in the process of this invention contain an acryl-functional organopolysiloxanes consisting of a plurality of units of the general formula

$$\underset{\substack{| \\ Z_b}}{R_aSi}O_{(4-a-b)/2} \qquad (I)$$

wherein Z is a reactive acryl-functional organic group, attached to silicon through an Si-C bond and bearing at least one group selected from acryloxy, methacryloxy or acrylamide groups. On average, at least two reactive Z groups per molecule of the organopolysiloxane are required to be within the scope of the present invention. Additionally, the organopolysiloxane must have an average of more than two of said acryl groups per molecule.

In the above formula, R is a non-reactive group which may be independently selected from alkyl radicals having 1 - 6 carbon atoms, such as methyl, ethyl, propyl, butyl, isopropyl or hexyl. The R group may also be selected from cycloaliphatic radicals, such as cyclopentyl, cyclohexyl and cyclooctyl radicals. Alternatively. R can be an aryl group such as phenyl, benzyl, styryl, tolyl and xenyl. Still further, R may be a monovalent halohydrocarbyl group having 1 to 6 carbon atoms such as 3,3,3-trifluoropropyl, 3-chloropropyl and perfluorobutylethyl. Finally, R may be a haloaromatic group, such as 2,3-dichlorophenyl. It is preferred that R is selected from methyl, phenyl or 3,3,3-trifluoropropyl radicals.

The non-reactive units of the organopolysiloxanes of this invention may be composed of any combination of siloxane units of the formulae $R_3SiO_{1/2}$, $R_2SiO_{2/2}$, $RSiO_{3/2}$, and $SiO_{4/2}$, bonded together by Si-O-Si bonds. Examples of suitable non-reactive siloxane units are endblocking triorganosiloxane units, such as $Me_3SiO_{1/2}$, $PhMe_2SiO_{1/2}$, $CF_3CF_2CF_2CF_2CH_2CH_2Me_2SiO_{1/2}$, $CF_3CH_2CH_2Me_2SiO_{1/2}$ and $Ph_2MeSiO_{1/2}$ ; backbone diorganosiloxane units, such as $Me_2SiO_{2/2}$, $PhMeSiO_{2/2}$, $CF_3CH_2CH_2MeSiO_{2/2}$, $Ph_2SiO_{2/2}$, $ClCH_2CH_2CH_2MeSiO_{2/2}$ and $CF_3CF_2CF_2CF_2CH_2CH_2MeSiO_{2/2}$; and branching monoorganosiloxane units, such as $MeSiO_{3/2}$, $PhSiO_{3/2}$ and $SiO_{4/2}$. Herein, Me denotes the methyl radical and Ph denotes the phenyl radical.

In any given organosiloxane unit of the liquid organopolysiloxane, the value of a may be 0, 1, 2 or 3, the value of b may be 0, 1 or 2 and the sum (a + b) is less than 4.

The exact nature of the organic portion of the Z group is not critical to the operability of this invention, but said organic portion must exclude functionality which would react with the continuous fluid phase or the acryl functionality. In other words, the organic portion of the Z groups serves only as a structure to link the acryl functionality thereof with the main body of the organopolysiloxane and is preferably chemically inert. In this regard, the term "inert" defines structures which will not interfere with the reaction between the acryl groups when exposed to ultraviolet radiation, as described below. Thus, for example, the organic portion of Z may be a divalent connecting group such as a hydrocarbon group having at least 3 carbon atoms or an arylene group, such as phenylene.

The acryl-functional siloxanes employed in the present invention are well known in the art and have been synthesized by various procedures. For example, acrylfunctional siloxane copolymers suitable for use in the present invention may contain the Z group -R‴OA wherein R‴ is a divalent hydrocarbon radical having 1 to 18 carbon atoms, or the corresponding oxyalkylene radical, and A is the radical

$$CH_2=C(B)\underset{|}{C}=O$$

in which B is hydrogen or methyl. Such copolymers may be prepared by methods described in United

States Patent No. 4,568,566 to Tolentino.

Another example of an acryl-functional siloxane copolymer suitable for use in the present invention contains the Z group

$$-R'(\underset{H}{N}C_2H_4)_kN(CH_2\underset{OH}{\underset{|}{CH}}(CH_2)_mOA)_2$$

wherein R' is a divalent hydrocarbon group, m is an integer between 1 and 10, k is 0, 1, 2, 3, or 4 and A has its previous meaning. Such copolymers may be prepared by methods described in United States Patent No. 4,293,397 to Sato et al. Briefly, these copolymers may be prepared by the addition of a glycidyl methacrylate to an amino-terminated diorganopolysiloxane.

Another example of an acryl-functional siloxane copolymer suitable for use in the present invention contains an acrylated urethane silicone having a Z group selected from

-DNHG or -DNGDNHG

wherein D is a divalent saturated hydrocarbon radical of from 1 to 6 carbon atoms and G is the radical

$$-CONHC_{m'}H_{2m'}OCO\underset{B}{\underset{|}{C}}=CH_2$$

In the above structure, B is selected from hydrogen or the methyl radical while m' can be 2, 3 or 4. The preparation of these acryl-functional siloxanes is described by Gornowicz et al. in U.S. Patent No. 4,563,539.

Yet another example of an acryl-functional siloxane copolymer suitable for use in the present invention contains the Z group taught in U.S. Patent No. 4,369,300 to Carter et al. which discloses the reaction of a silicone carbinol, a polyisocyanate and a hydroxyacrylate.

Acryl-functional siloxane copolymers suitable for use in the present invention may also be made by reacting an amine-functional silicone with a di-or multi-functional acryl-functional compound by a Michael-type addition reaction. These acryl-functional silicone compounds, and their preparation, are described in a copending application to Lee and Lutz, entitled "Acryl Functional Silicone Compounds," Serial No. 816,477, filed January 6, 1986.

Acrylamide-functional organopolysiloxanes suitable for use in the present invention contain groups having the structure

$$CH_2=C(B)\overset{-NR''''}{\overset{|}{C}}=O$$

wherein B is either hydrogen or methyl and R'''' represents hydrogen or a monovalent hydrocarbon radical such as methyl, ethyl or propyl. Examples of such acrylamide-functional organopolysiloxanes may be found in United States Patent No. 4,608,270 to Varaprath. In this case, the Z group has the structure

-QNAQ'NAR''''

wherein Q and Q' denote alkylene radicals having 2-6 carbon atoms. In the above formula, A again denotes the radical

$CH_2=C(B)\underset{|}{C}=O$ (Group A)

wherein B is hydrogen or methyl. Briefly, such siloxanes can be prepared by mixing an acyl halide with an aminosilicone compound having at least two silicon-bonded amino-substituted hydrocarbon radicals. The mixing step is carried out in the presence of an aqueous solution of an alkaline material and a water insoluble solvent for said aminosilicon compound.

These and other acryl-functional organopolysiloxanes known in the art may be employed for the purposes of this invention, provided they comply with the above-mentioned restrictions on reactivity.

Preferred acryl-functional organopolysiloxanes of this invention are selected from linear polymers having structures which may be represented by the average formulae

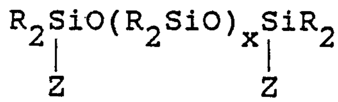

(II)

or

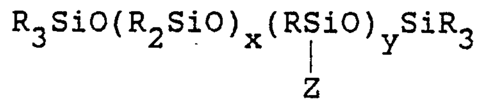

(III)

wherein R has the same meaning defined above, the average value of x may vary from about 5 to about 150 and the average value of y may vary from 2 to about 30. In formula (II), the acryl-functional groups are terminal to the siloxane chain and in formula (III) they are pendant to the chain.

For the purposes cf the present invention, a preferred Z group is Z′ which is represented by the formula

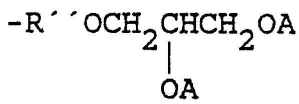

(Group Z′)

wherein R″ is a divalent hydrocarbon group having from 3 to 6 carbon atoms. Preferably, R″ is trimethylene. In the above formula. A is the same as previously defined.

Siloxanes bearing the group Z′ may be prepared according to the following synthesis steps. The synthesis is illustrated for the case of siloxanes having terminal acryl-functional groups, R″ being trimethylene and R being methyl, but the procedure applies equally to siloxanes having different R″ and pendant reactive groups.

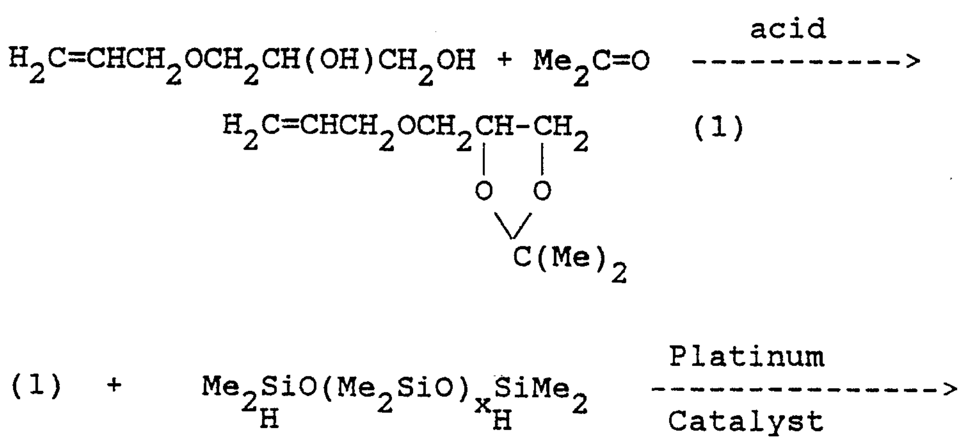

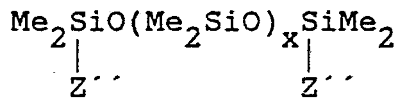

(2)

wherein Z´´ denotes

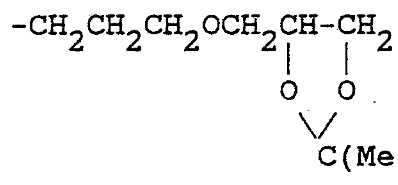

(Group Z´´)

MeOH/acid

(2) --------------->

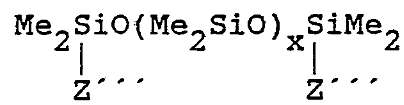

(3)

wherein Z´´´ denotes

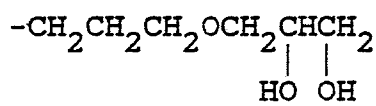

(Group Z´´´)

acid

(3) + $CH_2{=}CH_2C(O)OH$ ------>

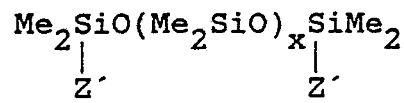

(4)

This preparation has been described in detail in a copending application by P. Lo, entitled "Dioxolane, Diol and Diacrylate Silicon Compounds and Method for Their Preparation and Use," Serial No. 914,899, filed on October 3, 1986 and assigned to the assignee of this invention.

Furthermore, the siloxane represented by formula (3), above, is known in the art and may alternatively be prepared by a method disclosed by Okazaki et al. in U.S. Patent No. 4,431,789.

The most preferred acryloxy-functional organopolysiloxanes of this invention have the structure

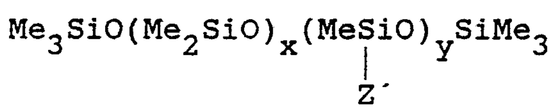

(IV)

or

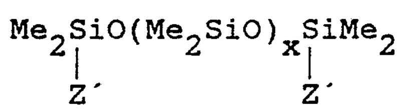

(V)

wherein Z′ has been defined, the average value of x may vary from about 5 to about 150 and the average value of y may vary from 2 to about 8. Such preferred acryloxy-functional methylpolysiloxanes are fluids having a viscosity between about 10 and 1000 cP at 25°C.

Similarly, the most preferred acrylamide-functional methylpolysiloxanes of this invention have the structure

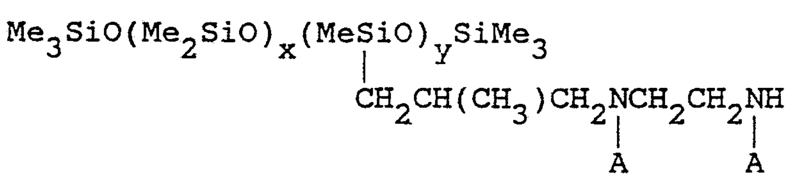

(VI)

in which A is the radical

$CH_2=C(B)\ \underset{|}{C}=O$

and B is hydrogen or methyl. Here the average value of x may vary from about 5 to 100 and the average value of y may vary from 2 to about 5. This acrylamide-functional organopolysiloxane may be prepared according to methods described by Varaprath, cited supra.

Any combination of reactive and non-reactive organosiloxane units, which is within the above-mentioned constraints, may be used for the purposes of the present invention, provided that the convertible organopolysiloxane compositions are liquid at room temperature, i.e., they flow. The initial viscosity of the liquid mixture is not critical and may range from about 10 to about 4000 cP at 25°C. A preferred viscosity of the convertible organopolysiloxane composition to be used for any particular combination of internal material and fluid continuous phase may be determined by routine experimentation.

When the organopolysiloxanes are free of $-SiO_{3/2}$ and $SiO_{4/2}$ siloxane units, and the total of all the reactive radicals does not exceed about 10 percent of all the radicals therein, the compositions of this invention generally form elastomeric microparticles when cured. Such is the case when the preferred acryl-functional organopolysiloxanes of this invention are employed. As the number of $-SiO_{3/2}$ and $SiO_{4/2}$ siloxane units and/or the percentage of reactive radicals therein are increased, more resinous microparticles are obtained.

The preferred embodiments of this invention result in elastomeric microparticles which are formed by curing the preferred convertible organopolysiloxane compositions. These compositions result when the organopolysiloxane is selected from the siloxanes represented by either formula (II) or formula (III), above, and contains up to about 15 mole percent acryl-functional siloxane units. Organopolysiloxanes represented by formula (IV), (V) or (VI) are most preferred for use in the process of this invention.

In addition to the organopolysiloxane, the convertible compositions of the present invention may contain inhibitors, solvents, pigments, dyes, stabilizers, extenders and plasticizers as well as other adjuvants commonly employed in the art.

The convertible organopolysiloxane compositions preferably contain a photosensitizer to decrease the time that is needed to convert the organopolysiloxane composition from the liquid to the solid state, under the influence of ultraviolet radiation. Photosensitizers are well known in the art and include, for example, acetophenone, benzophenone, propiophenone, xanthone, anthraquinone, fluorenone, 3-methylacetophenone, 3-bromoacetophenone, 4-methylacetophenone, benzaldehyde, carbazole, triphenylamine, 2-hydroxy-2-methyl-1-phenylpropane-1-one and 1-hydroxycyclohexylphenyl ketone. The amount of any particular photosensitizer to be used in this invention is merely an amount sufficient to photosensitize the system, as indicated by an improved rate of microparticle formation. Generally an amount of up to 5 percent by weight of photosensitizer, based on the total amount of the organopolysiloxane is sufficient.

The internal material, i.e. the material to be encapsulated by the process of this invention, may be any solid particle, liquid or gas which does not chemically react with the fluid continuous phase or the convertible organopolysiloxane composition or which does not dissolve extensively in the fluid continuous phase. The internal material should also not be adversely affected by ultraviolet radiation as used in this process.

Examples of suitable internal materials for this invention include corrosion inhibitors, adhesives, catalysts, colorants, cosmetics, curing agents, deodorants, detergents, drugs, enzymes, flavors, foods, fuels, inks, insecticides, metals, medicaments, monomers, fragrances, oils, pheromones, plasticizers, propellants, solvents, solid substrates containing an absorbed active component and vitamins.

The fluid continuous phase must be chemically unreactive with and not dissolve the convertible organopolysiloxane composition. Although it is not necessary, especially when preparing microcapsules having a dispersed internal material, it is preferred that the fluid continuous phase will not dissolve the internal material extensively. The fluid continuous phase must be at least partially, and preferably totally, transparent to the ultraviolet radiation employed in the curing step of this process. The fluid continuous phase may be a gas, but preferably it is a liquid of suitable viscosity to permit the forming and maintaining of the dispersion.

Examples of fluids that are suitable for use as the continuous phase in the method of this invention are air, nitrogen, steam, water, mineral oil and perfluorocarbons. Selection of a suitable match of internal material and fluid continuous phase should be made to satisfy the non-reactivity and non-solubility requirements noted above.

In a preferred embodiment of this invention, the fluid continuous phase is water which contains a dispersion-stabilizing amount of a surfactant of the oil-in-water type to aid in the formation of the dispersion and to minimize agglomeration of discrete entities and microparticles during the irradiation process. Said

surfactant may be the anionic type, such as salts of alkyl sulfates, salts of alkyl benzene sulfonates and salts of poly(oxyethylene)alkyl ethers, poly(oxyethylene)alkylphenol ethers, and poly(oxyethylene)alkyl esters. Preferably, any surfactant that is used is free of any groups which can react with the convertible organopolysiloxane composition. The proper amount of oil-in-water type surfactant to be used may vary widely and can be determined by simple experimentation. Generally, less than 5 percent by weight, based on the weight of water, is sufficient.

In the method of this invention, a dispersion consisting essentially of discrete entities, hereinafter further delineated, dispersed in a UV-transparent fluid continuous phase, is prepared and is simultaneously or subsequently exposed to ultraviolet radiation to convert the discrete entities to microparticles. Said dispersion may be prepared by any suitable method, such as stirring, homogenizing or emulsifying, which will provide a discontinuous phase of discrete entities which are maintained in the dispersed state while the dispersion is being exposed to ultraviolet radiation.

In one embodiment of this invention, which provides microspheres, the discrete entities consist essentially of spheres, up to about 5 mm in diameter, of a liquid organopolysiloxane composition which is convertible to the solid state by ultraviolet radiation. These discrete entities may be prepared by dispersing the liquid organopolysiloxane composition in the continuous phase fluid using any suitable method for dispersing a liquid in an incompatible fluid. These methods are well known in the art and need not be detailed here. Upon exposure to ultraviolet radiation these discrete entities experience a curing reaction which converts them to the solid state to provide microspheres. These microspheres, either elastomeric or resinous, are useful as filler particles in various fluid compositions such as greases, sealants and adhesives and as substrate particles in chromatography columns.

In another embodiment of this invention, which provides microcapsules, the discrete entities consist essentially of sphere-like particles, up to about 5 mm in diameter, having an internal material surrounded by a convertible organopolysiloxane composition. Upon exposure to ultraviolet radiation, the convertible organopolysiloxane composition is cured to the solid state thereby encapsulating the internal material and providing microcapsules. These microcapsules are useful as time release capsules, such as for the controlled release of herbicides, fertilizers and medicaments. However, the type of microcapsules that are produced by the method of this invention is determined by the manner in which the dispersion is prepared.

In a first manner for preparing the dispersion of discrete entities consisting essentially of an internal material surrounded by a convertible organopolysiloxane composition, the internal material to be microencapsulated is first dissolved or dispersed in the convertible organopolysiloxane composition and the resulting solution or dispersion is thereafter dispersed in the continuous phase fluid. In this manner, a major portion of microcapsules containing the internal material dissolved and/or dispersed throughout the solid organopolysiloxane is obtained after irradiation. When the internal material is insoluble in the liquid organopolysiloxane composition, there also may be obtained minor amounts of microcapsules containing a discrete core of internal material. To provide a maximum portion of microcapsules having a dispersed internal material, vigorous mixing of the internal material and the convertible organopolysiloxane composition should be used. In some cases, it may be desired or necessary to use a suitable surfactant to achieve proper dispersion of an internal material which is insoluble in the convertible organopolysiloxane composition.

In a second manner for preparing the dispersion of discrete entities consisting essentially of an internal material surrounded by a convertible organopolysiloxane composition, the internal material to be encapsulated is dispersed in the continuous phase fluid and the convertible organopolysiloxane composition is simultaneously, or subsequently, codispersed therewith. In this manner, a major portion of microcapsules containing the internal material localized as a discrete core in the solid organopolysiloxane is obtained after irradiation. There also may be obtained by this second manner minor amounts of microspheres of solid organopolysiloxane which are free of the internal material. For maximum yield of microcapsules having a discrete core of internal material, it is preferred to disperse the internal material and the convertible organopolysiloxane composition simultaneously in the fluid continuous phase, using moderate mixing such as stirring, rather than homogenizing or emulsifying.

The dispersion of discrete entities in fluid continuous phase may be exposed to ultraviolet radiation at any suitable time. Preferably, the dispersion is exposed to ultraviolet radiation as soon as it is formed and the exposure is continued until the convertible organopolysiloxane composition is converted to the desired state of solidification. Preferably, the dispersion is exposed until the microparticles have sufficient strength to permit isolation by standard methods, such as filtration and centrifugation, without fragmenting the solid organopolysiloxane. Further curing may be done if desired. In the case of shelf-stable dispersions, exposure thereof to ultraviolet radiation may be delayed, if desired.

The dispersion of discrete entities in fluid continuous phase may be irradiated in any of the well-known

manners such as by immersion of an electrically protected ultraviolet radiation source in the dispersion or by external exposure of the dispersion to a suitable source such as a mercury vapor lamp, an electric arc or the sun. Of course, the rate of conversion of discrete entities to microparticles is directly related to the intensity of ultraviolet radiation incident on the convertible organopolysiloxane composition and one should consider exposure parameters, such as the intensity of the ultraviolet source, its distance from the dispersion and the nature of the intervening space, when practicing the method of this invention. Ultraviolet radiation having wave lengths in the range of 200-400 nm is most effective.

In the method of this invention, the microparticles may be separated from or allowed to remain in the reaction mixture after irradiation, as desired. However, because of the pervious nature of organopolysiloxane elastomers and resins, microcapsules containing an internal material which is soluble in the fluid continuous phase should be separated from the fluid continuous phase as soon as they are formed or shortly thereafter to minimize any undesired leaching of the internal material by the fluid continuous phase.

The following examples are presented to further illustrate the process of this invention, but are not to be construed as limiting the invention, which is delineated in the appended claims. All parts and percentages in the examples are on a weight basis unless indicated to the contrary.

Example 1

Into a 2-liter flask, equipped with magnetic stirrer, water trap and condenser, was charged 195 grams of 3-allyloxy-1,2-propanediol, 225 grams of acetone and 480 grams of toluene. Four grams of concentrated sulfuric acid was then added to this mixture. This combination was stirred and heated to reflux, whereupon the water which formed was trapped over a 13 hour period. The reaction mixture was cooled to room temperature and neutralized with 50 grams of $NaHCO_3$. Distillation of the organic layer yielded 161 grams of a product having the formula

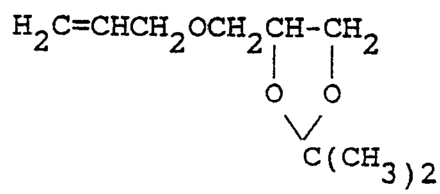

which product had a boiling point range of 57-58°C. at 4.5 mm Hg.

Example 2

Twenty grams of the product of Example 1 was mixed with 80 grams of an SiH-terminated siloxane having the average formula $H(Me_2)SiO(Me_2SiO)_{14}Si(Me_2)H$ wherein Me represents the methyl group. To this mixture was added 0.006 grams of a platinum catalyst which contained 4% platinum and was prepared according to the method described in Example 1 of United States Patent No. 3,419,593 to Willing. The mixture was stirred and reaction was evidenced by a mild exotherm whereupon the temperature rose to about 70°C. Reaction was completed by heating at 70°C. for an additional 5 hours, at which point the infrared absorption due to SiH (2170 cm-1) had disappeared. The product had the average structure

$Z''(Me_2)SiO(Me_2SiO)_{14}Si(Me_2)Z''$

wherein Z" is the group

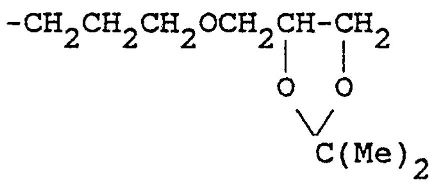

Example 3

To a mixture of 30 grams of methyl alcohol and 3 grams of concentrated hydrochloric acid was added 98 grams of the product of Example 2. This combination was heated up to a temperature of 74°C. to remove volatiles and further stripped at 80°C./30 mm Hg for about 30 minutes. The product was cooled to room temperature and filtered to yield 83.5 grams of a viscous fluid having the average structure

$Z'''(Me_2)SiO(Me_2SiO)_{14}Si(Me_2)Z'''$

wherein Z''' is

$-CH_2CH_2CH_2OCH_2CH(OH)-CH_2(OH)$

The theoretical hydroxyl content of 4% was confirmed as 4.0% +/-0.2%.

Example 4

A 50-ml flask, equipped with thermometer and condenser, was charged with 30 grams of a fluid prepared according to the method of Example 3, above, 6.2 grams of acryloyl chloride and 0.007 gram of hydroquinone. This mixture was heated at 150°C. for 30 minutes, during which time HCl was allowed to escape. The reaction mixture was cooled to room temperature, neutralized with 2.5 grams of calcium carbonate and filtered, resulting in an acrylate-functional polydimethylsiloxane having the average structure

$Z'(Me_2)SiO(Me_2SiO)_{14}Si(Me_2)Z'$

wherein Z' is the group

$-CH_2CH_2CH_2OCH_2CH(OA)-CH_2(OA)$

in which A is

$-C(=O)CH=CH_2$

Example 5

Into a flask equipped with a stirrer and thermometer, was charged 7.86 grams of the acrylate-functional polydimethylsiloxane of Example 4 and 31.0 grams of a blend of cyclic dimethylsiloxanes having about three to six siloxane units. To this mixture was added about 0.02 gram of trifluoromethane sulfonic acid and the reactants were stirred and heated at 70°C. for 3 hours. The resulting fluid was cooled to room temperature, neutralized with solid $CaCO_3$ (2 grams) and filtered. Presence of acrylate functionality was verified by infrared spectroscopy, the average structure of the fluid being

$Z'(Me_2)SiO(Me_2SiO)_{98}Si(Me_2)Z'$

wherein Z' is the group

$-CH_2CH_2CH_2OCH_2CH(OA)-CH_2(OA)$

in which A is

$-C(=O)CH=CH_2$

Example 6

Ten grams of the acryloxy-functional dimethylpolysiloxane of Example 4 was mixed with 0.5 gram of Irgacure® 500 (Ciba-Geigy Corp., Ardsley, NY). Irgacure® 500 is described as a 1:1 ratio of the photosensitizers benzophenone and 1-hydroxycyclohexylphenyl ketone. This mixture was dispersed in 400 cc of water containing 0.4 gram of octylphenoxypolyethoxy(40)ethanol (Triton® X-405, Rohm and Haas, Philadelphia, Pennsylvania). The dispersion so formed was stirred in a quartz reaction flask at a moderate rate, using a paddle stirrer, while it was irradiated with a 100 W ultraviolet medium pressure mercury vapor lamp placed approximately 10 mm from the flask. After irradiation for 5 minutes, the product was filtered, washed with water and dried. The resulting cured microspheres were clear and uniform in dimension, having a diameter of approximately 1 mm.

Example 7

Ten grams of the acryloxy-functional dimethylpolysiloxane of Example 5, above, was mixed with 0.5 gram of Irgacure® 500. This mixture was dispersed in 400 cc of water containing 0.4 gram of Triton® X-405 and cured by exposure to ultraviolet radiation in the manner described in Example 6, above. Uniform, slightly cloudy spheres, having a diameter of about 1 mm, resulted.

Example 8

An acrylamide-functional siloxane fluid having the average structure

$$Me_3SiO(Me_2SiO)_{69.3}(\underset{\underset{Y}{\vert}}{MeSiO})_{3.8}SiMe_3$$

wherein Y is the group

$$-CH_2CH(CH_3)CH_2\underset{\underset{A}{\vert}}{N}CH_2CH_2\underset{\underset{A}{\vert}}{NH},$$

in which A is the radical

$$CH_2=CH\underset{\vert}{C}=O,$$

was prepared according to the method of Example 6 of United States Patent No. 4,608,270 to Varaprath. Ten grams of this acrylamidefunctional dimethylpolysiloxane was mixed with 0.5 gram of a photosensitizer having the structure

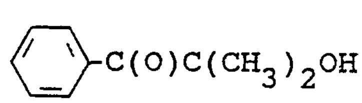

(Darocure® 1173, E M Industries, Hawthorne, NY). This mixture was dispersed in 400 cc of water containing 5 grams of octylphenoxypolyethoxy(10)ethanol (Triton® X-100, Rohm and Haas, Philadelphia, Pennsylvania). The dispersion was cured by exposure to ultraviolet radiation in the manner described in Example 6, above, wherein the dispersion was subjected to rapid stirring fof 30 minutes during the irradiation. A fine white powder resulted, the particles having a dimensional range of about 0.01 to 0.1 mm.

Example 9

Ten grams of the acrylamide-functional dimethylpolysiloxane of Example 8 was mixed with 0.5 gram of Darocure® 1173. This mixture was dispersed in 400 cc of water containing 0.4 gram of Triton® X-405 and cured by exposure to ultraviolet radiation in the manner described in Example 6, above, using moderate stirring rates for 5 minutes. Clear, hard spheres, having a diameter in the range of 3 - 5 mm, were obtained.

Example 10

A blend of 5.0 grams of the acrylate-functional dimethylpolysiloxane of Example 4 and 0.25 gram of Irgacure® 500 was prepared. Three grams of finely powdered aspirin was dispersed in the blend. This dispersion was then added to 400 cc of water containing 0.5 gram of Triton® X-405 in a quartz reaction flask and cured for 15 minutes in the manner described in Example 6. After irradiation, the product was filtered, washed and dried to recover 7.4 grams of silicone microcapsules containg the aspirin and measuring approximately 0.5-1 mm in diameter.

Example 11

A blend of 5.0 grams of the acrylate-functional dimethylpolysiloxane of Example 5 and 0.25 gram of Irgacure® 500 was prepared. Three grams of finely powdered aspirin was dispersed in the blend. This dispersion was dispersed in water, cured and recovered, according to the method described in Example 10 to yield 7.0 grams of microcapsules containing the aspirin. These particles had a diameter in the range of about 1-3 mm.

Example 12

A blend of 5.0 grams of the acrylamide-functional dimethylpolysiloxane of Example 8 and 0.25 gram of Darocure® 1173 was prepared. Two grams of finely powdered aspirin was dispersed in the blend. This dispersion was dispersed in water, cured and recovered according to the method described in Example 10 to yield 6.8 grams of encapsulated aspirin in particles having a diameter in the range of about 0.5-1.0 mm.

Example 13

Twenty-five grams of an acrylamide-functional siloxane having a structure similar to that shown in Example 8, above, but having 5 mole percent methyl/acrylamide-functional siloxane units and a degree of polymerization of about 75, was mixed with 1.25 grams of Darocure® 1173. This mixture was added to 400 ml of distilled water and 10 grams of a fragrance oil, 46.113/E, was added. The fragrance oil, containing olefinic unsaturation, (46.113/E; Eurand America, Inc., Vandalia, Ohio) is described as a perfume oil having an herbal odor, a specific gravity of 1.021-1.041, a flash point of 167°C. and being slightly soluble in water. Triton® X100 (0.5 gram) was added and the so formed dispersion was stirred at 800 r.p.m. and irradiated for 25 minutes as in the other examples. After washing and filtering, the dried particles were hard and tack-free, having a bimodal size distribution in the range of about 0.5 mm diameter.

(Comparative) Example 14

According to methods disclosed in United States Patent No. 4,370,160, cited supra, 20 grams of a mercaptofunctional silicone having the average structure

$$Me_3SiO(Me_2SiO)_{113}(\underset{\displaystyle CH_2CH_2CH_2SH}{\underset{|}{MeSiO}})_{10}SiMe_3$$

was mixed with 40 grams of an olefin-functional silicone having the average structure

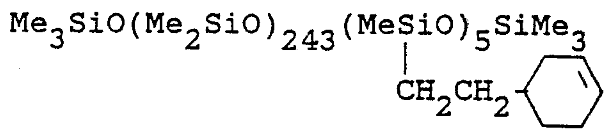

and 0.9 gram of the photosensitizer benzophenone. This blend was dispersed in 400 ml of water containing 5 grams of Triton® X100 The so formed dispersion was stirred in a reaction tube and irradiated for 5 minutes using moderate stirring rates, in a manner similar to the previous examples. Uniform particles, having a diameter of about 1 mm, were obtained. However, the particles, when isolated, were still sticky to the touch.

<u>(Comparative) Example 15</u>

The fragrance oil used in Example 13, above, was encapsulated according to the methods described in United States Patent No. 4,370,160. Each of the silicone reactants of (Comparative) Example 14 was blended with benzophenone, such that the latter compound constituted 1.5% of each respective blend. A homogeneous mixture of 8.33 grams of the mercapto-functional silicone blend with 16.66 grams of the olefin-functional silicone blend was prepared. This mixture was added to a reaction flask containing 25 grams of the fragrance oil dispersed in 400 ml of water containing 0.4 gram of octylphenoxypolyethoxy(40)-ethanol (Triton® 405, Rohm and Haas, Philadelphia, Pennsylvania). This dispersion was stirred in the reaction flask and irradiated with a UV medium pressure mercury vapor lamp placed approximately 10 mm from the flask. After 1 hour of irradiation, little cure was evident; the particles, when isolated, were still sticky to the touch and incompletely cured. This observation illustrates the difficulty of forming a UV-cured encapsulation system when the material to be encapsulated interferes with a mercapto-vinyl cure.

## Claims

1. A process for preparing microspheres of solid organopolysiloxane, said process comprising:

(I) preparing a dispersion of discrete entities in a fluid continuous phase by dispersing, in the continuous phase fluid, a liquid organopolysiloxane composition, convertible by ultraviolet radiation to the solid state, said fluid continuous phase being transparent to ultraviolet radiation and said liquid organopolysiloxane composition being insoluble in the fluid continuous phase and consisting essentially of an organopolysiloxane having attached thereto through silicon-carbon bonds an average of at least two Z groups per molecule, wherein Z is a monovalent organic moiety containing at least one acryl group selected from acryloxy, methacryloxy or acrylamide groups, said organopolysiloxane having an average of more than two of said acryl groups per molecule; and

(II) exposing the dispersion of (I) to ultraviolet radiation until the liquid organopolysiloxane composition is converted to the solid state.

2. The process according to claim 1, wherein said liquid organopolysiloxane composition, convertible by ultraviolet radiation to the solid state, further contains a photosensitizing amount of a photosensitizer.

3. The process according to claim 2, wherein said fluid continuous phase is water comprising a dispersion-stabilizing amount of an oil-in-water surfactant.

4. The process according to claim 3, wherein said organopolysiloxane is selected from linear polymers having the average structure

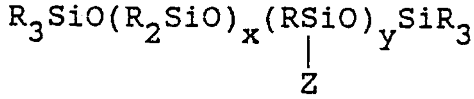

or

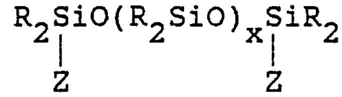

wherein R is selected from alky radicals having 1-6 carbon atoms, cycloaliphatic radicals, aryl groups, halohydrocarbyl groups having 1-6 carbon atoms or haloaromatic groups, the average value of x can vary from about 5 to about 150 and the average value of y can vary from 2 to about 30.

5. The process according to claim 4, wherein said Z group is represented by the formula
-QNAQ'NAR''''
in which Q and Q' denote alkylene radicals having 2-6 carbon atoms, R'''' is hydrogen or methyl and A is the group
$CH_2{=}C(B)\underset{|}{C}{=}O$
where B is selected from hydrogen or the methyl radical.

6. The process according to claim 4, wherein said Z group is represented by the formula

$$-R''OCH_2\underset{\substack{|\\ OA}}{C}HCH_2OA$$

in which R'' is a divalent hydrocarbon group having 3 to 6 carbon atoms and A is the group
$CH_2{=}C(B)\underset{|}{C}{=}O$
where B is selected from hydrogen or the methyl radical.

7. A process for preparing microcapsules of an internal material localized as a core in a solid organopolysiloxane, said process comprising:

(I) preparing a dispersion of discrete entities in a fluid continuous phase by dispersing the internal material in the continuous phase fluid and simultaneously or subsequently codispersing therewith a liquid organopolysiloxane composition convertible by ultraviolet radiation to the solid state and insoluble in the fluid continuous phase, said fluid continuous phase being transparent to ultraviolet radiation and said organopolysiloxane composition consisting essentially of an organopolysiloxane having attached thereto through silicon-carbon bonds an average of at least two Z groups per molecule, wherein Z is a monovalent organic moiety containing at least one acryl group selected from acryloxy, methacryloxy or acrylamide groups, said organopolysiloxane having an average of more than two of said acryl groups per molecule; and

(II) exposing the dispersion of (I) to ultraviolet radiation until the liquid organopolysiloxane composition is converted to the solid state.

8. The process according to claim 7, wherein said liquid organopolysiloxane composition, convertible by ultraviolet radiation to the solid state, further contains a photosensitizing amount of a photosensitizer.

9. The process according to claim 8, wherein said fluid continuous phase is water comprising a dispersion-stabilizing amount of an oil-in-water surfactant.

10. The process according to claim 9, wherein said organopolysiloxane is selected from linear copolymers having the average structure

$$R_3SiO(R_2SiO)_x(\underset{\substack{|\\ Z}}{RSiO})_ySiR_3 \quad \text{or} \quad R_2\underset{\substack{|\\ Z}}{Si}O(R_2SiO)_x\underset{\substack{|\\ Z}}{Si}R_2$$

wherein R is selected from alky radicals having 1-6 carbon atoms, cycloaliphatic radicals, aryl groups, halohydrocarbyl groups having 1-6 carbon atoms or haloaromatic groups, the average value of x can vary from about 5 to about 150 and the average value of y can vary from 2 to about 30.

11. The process according to claim 10, wherein said Z group is represented by the formula
-QNAQ'NAR''''
in which Q and Q' denote alkylene radicals having 2-6 carbon atoms, R'''' is hydrogen or a methyl and A is the group
$CH_2{=}C(B)\underset{|}{C}{=}O$
where B is selected from hydrogen or the methyl radical.

12. The process according to claim 10, wherein said Z group is represented by the formula

$$-R''OCH_2\underset{\substack{|\\ OA}}{C}HCH_2OA$$

in which R'' is a divalent hydrocarbon group having 3 to 6 carbon atoms and A is the group

$CH_2=C(B)\ C=O$
|

where B is selected from hydrogen or the methyl radical.

13. A process for preparing microcapsules of an internal material dispersed throughout a solid organopolysiloxane, said process comprising:

(I) preparing a dispersion of discrete entities in a fluid continuous phase by dispersing or dissolving the internal material in a liquid organopolysiloxane composition, convertible by ultraviolet radiation to the solid state, and dispersing the resulting dispersion or solution in the continuous phase fluid, said fluid continuous phase being transparent to ultraviolet radiation and said organopolysiloxane composition being insoluble in the fluid continuous phase and consisting essentially of an organopolysiloxane having attached thereto through silicon-carbon bonds an average of at least two Z groups per molecule, wherein Z is a monovalent organic moiety containing at least one acryl group selected from acryloxy, methacryloxy or acrylamide groups, said organopolysiloxane having an average of more than two of said acryl groups per molecule; and

(II) exposing the dispersion of (I) to ultraviolet radiation until the liquid organopolysiloxane composition is converted to the solid state.

14. The process according to claim 13, wherein said liquid organopolysiloxane composition, convertible by ultraviolet radiation to the solid state, further contains a photosensitizing amount of a photosensitizer.

15. The process according to claim 14, wherein said fluid continuous phase is water comprising a dispersion-stabilizing amount of an oil-in-water surfactant.

16. The process according to claim 15, wherein said organopolysiloxane is selected from linear copolymers having the average structure

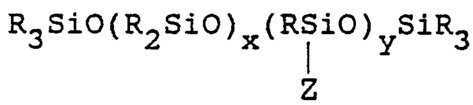

or

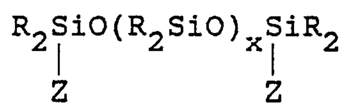

wherein R is selected from alkyl radicals having 1-6 carbon atoms, cycloaliphatic radicals, aryl groups, halohydrocarbyl groups having 1-6 carbon atoms or haloaromatic groups, the average value of x can vary from about 5 to about 150 and the average value of y can vary from 2 to about 30.

17. The process according to claim 16, wherein said Z group is represented by the formula

-QNAQ'NAR''''

in which Q and Q' denote alkylene radicals having 2-6 carbon atoms. R'''' is hydrogen or a methyl and A is the group

$CH_2=C(B)\ C=O$
|

where B is selected from hydrogen or the methyl radical.

18. The process according to claim 16, wherein said Z group is represented by the formula

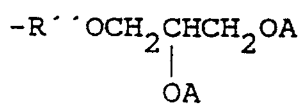

in which R'' is a divalent hydrocarbon group having 3 to 6 carbon atoms and A is the group

$CH_2=C(B)\ C=O$
|

where B is selected from hydrogen or the methyl radical.

19. The process according to claim 7 or 13, wherein said internal material is selected from enzymes, fragrances, corrosion inhibitors, catalysts or medicaments.

European Patent Office

# EUROPEAN SEARCH REPORT

Application Number

EP 87 30 9068

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | US-A-4 370 160 (M.J. ZIEMELIS)<br>* Column 17, lines 23-49 *<br>----- | 1 | B 01 J 13/02<br>C 08 J 3/03<br>C 08 J 3/28<br>C 08 L 83/04 |
| | | | **TECHNICAL FIELDS SEARCHED (Int. Cl.4)** |
| | | | B 01 J<br>C 08 J<br>A 61 K<br>B 41 M<br>C 08 L |

The present search report has been drawn up for all claims

| Place of search | Date of completion of the search | Examiner |
|---|---|---|
| THE HAGUE | 19-01-1988 | KERRES P.M.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document